# EUROPÄISCHE PATENTSCHRIFT

(11) **EP 0 786 981 B1**
(45) Veröffentlichungstag und Bekanntmachung des Hinweises auf die Patenterteilung: **16.09.1998**
(21) Anmeldenummer: 95935937.3
(22) Anmeldetag: 14.10.1995
(51) Int. Cl.: A61J 1/16, F25D 25/00, A01N 1/02, F28F 13/18

(54) **FRIERCONTAINER**
DEEP-FREEZING CONTAINER
CONTENEUR CONGELATEUR

(30) Priorität: 17.10.1994 DE 4437091
(43) Veröffentlichungstag der Anmeldung: 06.08.1997
(73) Patentinhaber: Sputtek, Andreas, 52070 Aachen (DE); Mingers, Bernd, 47877 Willich (DE)
(72) Erfinder: Sputtek, Andreas, 52070 Aachen (DE); Mingers, Bernd, 47877 Willich (DE)
(74) Vertreter: Luderschmidt, Schüler & Partner GbR
(86) Internationale Anmeldenummer: EP9504048
(87) Internationale Veröffentlichungsnummer: WO9611663

(56) Entgegenhaltungen:
- FR-A- 2 219 886
- US-A- 4 018 911
- US-A- 4 074 753
- US-A- 4 288 897
- US-A- 4 371 034
- US-A- 5 320 119

## Beschreibung

Die vorliegende Erfindung betrifft einen Behälter, der zum Tieffrieren von in elastischen Beutein befindlicher Suspension lebender Zellsubstanz geeignet ist.

Das Tiefgefrieren ermöglicht das Haltbarmachen lebender biologischer Zellen für praktisch unbegrenzte Zeit. So wird beispielsweise vor blutverlustreichen, zeitlich planbaren Eingriffen dem Patienten Blut entnommen, aufbereitet und anschließend tiefgefroren und gelagert. Damit ist es möglich, ein ausreichendes Depot mit Eigenblut des Patienten anzulegen, so daß dieser während der Operation und danach mit Eigenblut versorgt werden kann. Damit wird eine Infektion des Patienten mit im fremden Spenderblut möglicherweise enthaltenen und aufgrund des "diagnostischen Fensters" nicht nachweisbaren Hepatitiden und HIV verursachenden Viren zuverlässig verhindert. Desweiteren ermöglicht das allgemein als Kryokonservierung bezeichnete Tiefgefrieren die Bereitstellung von "Quarantäne-Präparaten" aus Fremdblutspenden, die Überbrückung temporärer Engpässe und die Bevorratung seltener Blutgruppen.

Üblicherweise wird die entnommene Vollblutkonserve durch Zentrifugation in Erythrozyten und Blutplasma aufgetrennt, das dann in tieftemperaturbeständige Beutel überführt wird. Diese werden anschließend in Metallbehälter eingelegt, mit denen sie zum Abwühlen in flüssigen Stickstoff eingetaucht werden. Damit die lebenden Zellen beim Gefrieren nicht geschädigt oder zerstört werden, ist bei Erythrozyten ein kontrolliertes und schnelles Abkühlen mit einer hohen Kühlrate notwendig. Die herkömmlichen Metallbehälter haben jedoch den Nachteil, daß sie nur einen begrenzten Wärmeübergang an das Kältemittel ermöglichen, so daß es bei nicht zu vermeidenden geometrischen Inhomogenitäten des in dem Behälter enthaltenen Beutels zu unkontrolliertem Abkühlen zumindest in diesen Teilbereichen kommt.

Vor diesem Hintergrund ist es daher Aufgabe der vorliegenden Erfindung, einen Behälter zum Tieffrieren von in elastischen Beuteln befindlicher Suspension lebender Zellsubstanz zur Verfügung zu stellen, mit dem ein schnelles und kontrolliertes Abkühlen der Zellsubstanz möglich ist.

Gelöst wird diese Aufgabe durch eine an der Außenseite des Behälters mittels einer Klebeschicht befestigte mikroporöse Schicht.

Diese mikroporöse Schicht auf der Außenseite des Behälters erhöht den Wärmeübergang an das Kältemittel und vergrößert dadurch erheblich die Kühlrate, so daß ein schnelles und kontrolliertes Abkühlen der Zellsubstanz möglich ist und damit die Überlebensrate der mit dem erfindungsgemäßen Behälter tiefgefrorenen Zellsubstanz deutlich gesteigert wird.

Gemäß einer bevorzugten Weiterbildung der Erfindung besteht die mikroporöse Schicht aus textilen Materialien. Diese können beispielsweise als textiles Klebeband aus Acetatseide mit einer temperaturbeständigen Arcrylatklebemasse ausgebildet werden, so daß das textile Material einfach und zuverlässig an der Außenseite des Behälters befestigt werden kann und dauerhaft schnellem Abkühlen und Wiedererwärmen standhält.

Eine besonders vorteilhafte Weiterbildung der Erfindung sieht vor, daß der Behälter zwei planparallel angeordnete dünnwandige metallische Platten, die die Behälterwandung bilden, und einen aus zwei Hälften bestehenden Rahmen aufweist, wobei zweckmäßigerweise sowohl die Platten als auch der Rahmen aus tieftemperatur-unempfindlichem Material bestehen. Vorteilhafterweise ist dabei jeweils eine Platte in einer Rahmenhälfte befestigt, die beiden Rahmenhälften sind mittels mindestens eines Gelenkes verschwenkbar aneinander befestigt und die in den Rahmenhälften befestigten Platten sind durch eine Arretierungsvorrichtung in der planparallelen Stellung arretierbar. Vorteilhafterweise weisen die in der planparallelen Stellung arretierten Platten dabei einen definierten Abstand voneinander auf, so daß der in den Behälter eingelegte Beutel beim Schließen des Behälters zu einer plattenförmigen Geometrie mit der erforderlichen Schichtdicke gepreßt wird. Damit prägt der Behälter dem elastischen Beutel eine im wesentlichen homogene Plattenform auf, so daß einerseits ein großes Oberflächen-/Volumenverhältnis erreicht wird und andererseits geometrische Inhomogenitäten wie Falten oder Auswölbungen vermieden werden. Dadurch wird ein weiter verbesserter Wärmeübergang erzielt, und ein unkontrolliertes Abkühlen durch geometrische Inhomogenitäten wird zuverlässig verhindert. Außerdem ermöglicht die dünne homogene Plattenform des Beutels eine platzsparende Lagerung und ein schnelles Wiedererwärmen.

Durch diese erfindungsgemäße Ausbildung des Behälters wird außerdem eine einfache und schnelle Handhabung ermöglicht, da der Behälter zum Einlegen und Entnehmen des elastischen Beutels mit der Suspension vollständig aufgeklappt werden kann.

In einer weiteren Ausführungsform ist in einer Rahmenhälfte eine Dichtung angeordnet, die vorteilhafterweise als Weichstoffdichtung ausgebildet ist und tieftemperaturbeständig ist, so daß der Behälterinnenraum flüssigkeitsdicht abdichtbar ist. Diese Dichtung kann aus PTFE oder einem anderen geeigneten Material bestehen

Da die elastischen Beutel, welche die Suspension aufnehmen, üblicherweise Anschlußstutzen und Pilotröhrchen aufweisen, sind vorteilhafterweise an mindestens einer der Platten eine oder mehrere Ausnehmungen angeordnet, die so ausgebildet sind, daß sie Anschlußstutzen oder Pilotröhrchen aufnehmen können. Damit behindern diese nicht das Schließen des Behälters, und der definierte planparallele Abstand der beiden Platten ist auch bei Verwendung eines elastischen Beutels mit Anschlußstutzen gewährleistet. Vorteilhafterweise sind diese Ausnehmungen mit einem Verschlußmittel flüssigkeitsdicht verschließbar, so daß das Kältemittel nicht in den Behälterinnenraum gelangen kann.

Eine weitere Ausführungsform sieht vor, daß die Platten auf ihrer zur Behälterinnenseite gerichteten Seite eine Oberflächenrauhigkeit von < 1 µm aufweisen, so daß der thermische Kontakt zwischen Beutel und Platten während des gesamten Abkühlvorganges gewährleistet ist.

Die Erfindung wird im folgenden anhand der Figuren näher erläutert. Dabei zeigt:
- Figur 1: eine Draufsicht auf den Behälter,
- Figur 2: eine Seitenansicht des Behälters in Richtung des Pfeils X in Figur 1, und
- Figur 3: einen Schnitt durch den Behälter entlang der Linie A-A in Figur 1.

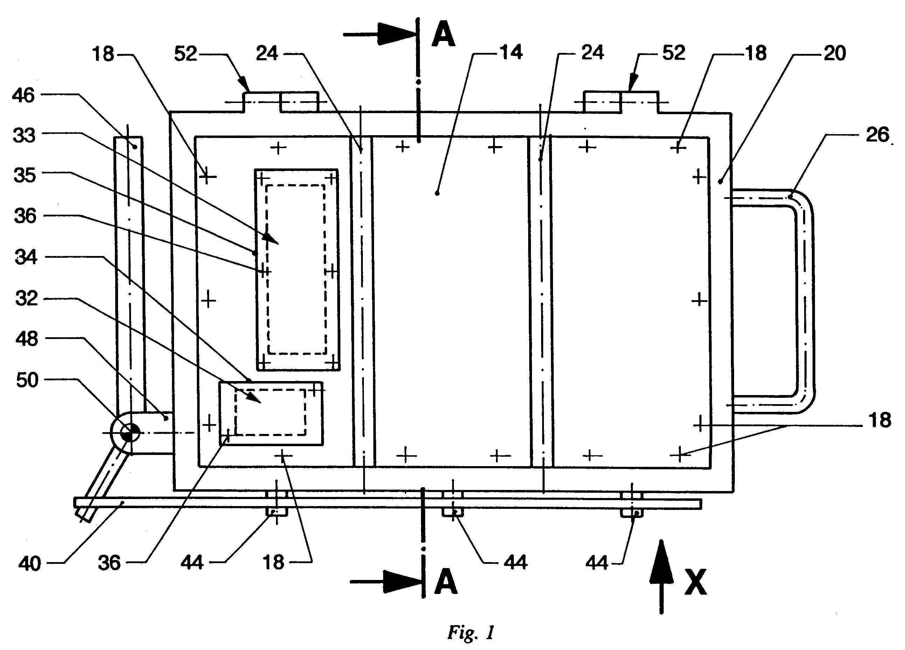

In Figur 1 ist eine Draufsicht des Behälters dargestellt. Dieser besteht im wesentlichen aus einer oberen Platte und einer unteren Platte, sowie aus einem zweiteiligen Rahmen, der aus einer oberen Rahmenhälfte und einer unteren Rahmerhälfte besteht. In dieser Ansicht ist jedoch nur die obere Platte 14 sowie die obere Rahmenhälfte 20 erkennbar. Die obere Platte 14 ist mit den Schrauben 18 an der oberen Rahmenhälfte 20 befestigt. Diese obere Rahmenhälfte 20 ist mit zwei Gelenken 52 verschwenkbar mit der nicht dargestellten unteren Rahmenhälfte verbunden und weist zwei Versteifungselemente 24 auf, die jeweils bei dem hier dargestellten Ausführungsbeispiel aus einem Rundstab bestehen. Diese Versteifungselemente 24 begrenzen die zwangsläufig beim Gefrieren der Suspension auftretente Ausbeulung der Platten 14, 16 auf einen definierten Wert. An der oberen Rahmenhälfte 20 ist außerdem ein Griff 26 befestigt, sowie Haltenasen 44. Diese Haltenasen 44 werden zur Verriegelung des Behälters zusammen mit den auf der unteren Rahmenhälfte angeordneten, identischen Haltenasen 44 von einer Arretierungsvorrichtung ergriffen. Diese besteht aus einem Hebel 46 und einer Kulisse 40. Der Hebel 46 der Arretierungsvorrichtung ist mit einem Bolzen 50 drehbar in einem an der unteren Rahmenhälfte befestigten Bock 48 gelagert. Schließlich weist die obere Platte 14 noch zwei Ausnehmungen 32 und 33 auf, die mit jeweils einer als Kappe ausgebildeten Abdeckung 34 bzw. 35 versehen sind. Die Abdeckungen 34 und 35 sind mit den Schrauben 36 mit der oberen Platte 14 verbunden. Bei diesem Ausführungsbeispiel nimmt die Ausnehmung 32 einen Anschlußstutzen (nicht dargestellt) auf, während die Ausnehmung 33 Pilotröhrchen zur Blutgruppenbestimmung aufnimmt, die ebenfalls nicht dargestellt sind.

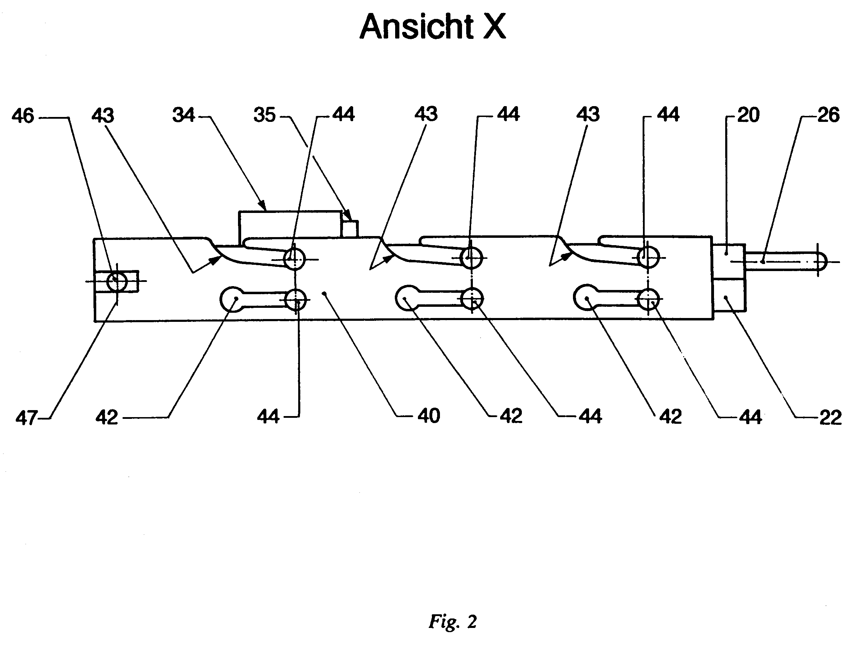

In Figur 2 ist eine Seitenansicht des Behälters in Richtung des Pfeils X in Figur 1 dargestellt. Wie hier gut zu erkennen ist, weist die Kulisse 40 eine Reihe von oberen Halteschlitzen 42 und einer Reihe von unteren Halteschlitzen 43 auf. Diese Halteschlitze ergreifen die Haltenasen 44 der oberen Rahmenhälfte 20 und der unteren Rahmenhälfte 22, wenn diese aufeinanderliegen. Die unteren Halteschlitze 42 sind dabei allseits geschlossen, während die oberen Halteschlitze 43 nach oben hin geöffnet sind. Die Flanken der Halteschlitze 43 weisen mit einer leichten Neigung nach oben zu ihrer Öffnung hin. Der Hebel 46 ist mit einem durch die strichpunktierte Linie 47 angedeuteten Bolzen mit der Kulisse 40 drehbar verbunden. Ebenfalls gut zu erkennen ist hier der an der oberen Rahmenhälfte 20 angeordnete Griff 26 sowie die als Kappe ausgebildete Abdeckung 34, die die in der oberen Plattenhälfte 14 angeordnete Ausnehmung 32 verschließt.

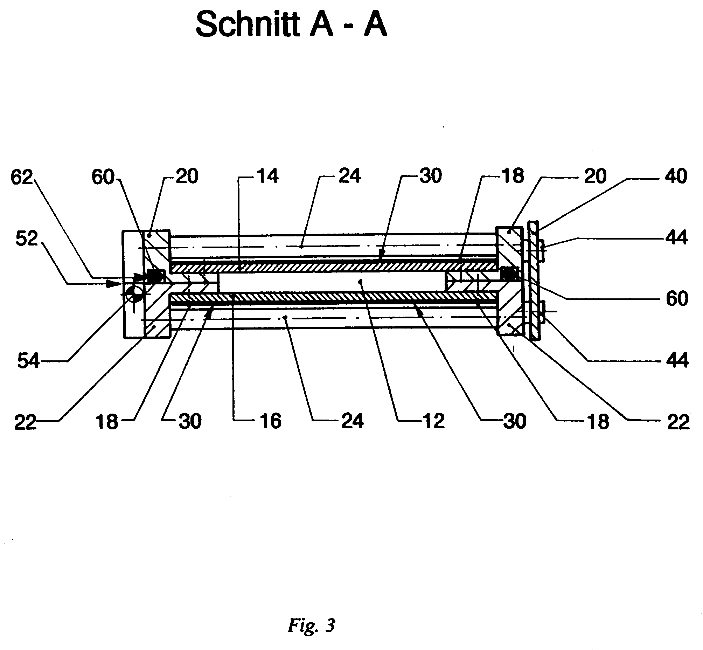

In Figur 3 ist ein Schnitt durch den Behälter entlang der Linie A-A der Figur 1 dargestellt. Die die Behälterwandungen bildende obere Platte 14 und untere Platte 16 sind jeweils mit durch die Linien 18 angedeuteten Schrauben mit der oberen Rahmenhälfte 20 bzw. der unteren Rahmenhälfte 22 verbunden. Auf der Außenseite sowohl der oberen Platte 14 als auch der unteren Platte 16 ist jeweils eine mikroporöse Schicht 30 aufgebracht, die bei dem hier dargestellten Beispiel aus einem textilen Material besteht, das mit einer tieftemperaturbeständigen Klebeschicht an der Außenseite der oberen Platte 14 und der unteren Platte 16 befestigt ist. In der oberen Rahmenhälfte 20 ist eine Nut 62 eingearbeitet, in der eine Dichtung 60 angeordnet ist. Diese ist bei dem hier beschriebenen Ausführungsbeispiel als tieftemperaturbeständige Weichstoffdichtung ausgebildet und dichtet den zwischen der oberen Platte 14, der unteren Platte 16, der oberen Rahmenhälfte 20 und der unteren Rahmenhälfte 22 gebildeten Behälterinnenraum 12 flüssigkeitsdicht ab. Die obere Rahmenhälfte 20 und die untere Rahmenhälfte 22 weisen jeweils Versteifungselemente 24 auf und sind durch die in den Scharnieren 52 angeordneten Bolzen 54 drehbar miteinander verbunden. Die Scharniere 52 und die Bolzen 54 sind dabei vorzugsweise aus Material mit im wesentlichen gleichen Ausdehnungskoeffizienten ausgebildet, so daß ein Klemmen vermieden wird und die Beweglichkeit des Gelenks auch bei tiefen Temperaturen gewährleistet ist. Die an der oberen Rahmenhälfte 20 und der unteren Rahmenhälfte 22 angeordneten Haltenasen 44 werden im geschlossenen Zustand des Behälters von der Kulisse 40 ergriffen. Der Behälter wird in der geschlossenen Stellung durch Selbsthemmung zwischen den schrägen Flanken der Halteschlitze 42 bzw. 43 und den Haltenasen 44 arretiert.

Die Funktionsweise des erfindungsgemäßen Behälters wird im folgenden unter Bezugnahme auf die oben ausführlich beschriebenen Figuren 1 bis 3 erläutert. Die zum Tieffrieren vorgesehene Suspension lebender Zellsubstanz wird in einen elastischen, zum Tieffrieren geeigneten Beutel gefüllt und dieser Beutel dann in den Behälterinnenraum 12 eingelegt. Dazu wird die obere Rahmenhälfte 20 nach oben weggeklappt, so daß der Behälterinnenraum 12 zugänglich wird. In diesen wird dann der Beutel eingelegt und anschließend wieder die obere Rahmenhälfte 20 nach unten verschwenkt, bis sie auf der unteren Rahmenhälfte 22 aufliegt. Die obere Platte 14 und die untere Platte 16 sind dabei jeweils so in den zugehörigen Rahmenhälften 20 bzw. 22 befestigt, daß sie im geschlossenen Zustand des Behälters mit geringem Abstand planparallel zueinander angeordnet sind. Beim Schließen des Behälters wird dadurch der eingelegte Beutel zu einer plattenförmigen Geometrie mit einer geringen definierten Schichtdicke gepreßt, wobei er den Behälterinnenraum 12 annähernd vollständig ausfüllt und an der Innenseite der oberen Platte 14 und an der Innenseite der unteren Platte 16 anliegt. Dadurch werden geometrische Inhomogenitäten wie Falten oder Auswölbungen vermieden, so daß der Beutel eine im wesentlichen homogene Plattenform aufweist und damit ein großes Oberflächen-/Volumenverhältnis erreicht wird. In Verbindung mit der an der Außenseite der Platten 14 und 16 angebrachten mikroporösen Schicht 30, die den Wärmeübergang an das den Behälter umgebende Kältemittel deutlich erhöht, wird damit ein schnelles und kontrolliertes Abkühlen der Zellsubstanz möglich, so daß die Überlebensrate der mit dem erfindungsgemäßen Behälter tiefgefroren Zellsubstanz deutlich gesteigert wird.

Die an den Rahmerhälften 20 und 22 angeordneten Versteifungselemente 24 begrenzen die zwangsläufig beim Gefrieren der Suspension auftretende Ausbeulung der Platten 14 und 16, so daß die homogene Plattenform des im Behälter befindlichen elastischen Beutels im wesentlichen gewährleistet ist.

Nachdem der Beutel in den Behälterinnenraum 12 eingelegt und die obere Rahmenhälfte 20 auf die untere Rahmenhälfte 22 geschwenkt wurde, werden die obere Rahmenhälfte 20 und die untere Rahmenhälfte 22 durch die Kulisse 40 miteinender verriegelt. Die Kulisse 40 ist durch die allseits geschlossenen Halteschlitze 42 ständig mit der unteren Rahmenhälfte 22 verbunden, auf der sie aber durch die schlitzförmige Ausbildung der Halteschlitze 42 hin- und hergeschoben werden kann. Beim Herunterschwenken der oberen Rahmenhälfte 20 auf die untere Rahmenhälfte 22 gelangen die an der oberen Rahmenhälfte 20 angeordneten Haltenasen 44 in die nach oben offenen oberen Halteschlitze 43 der Kulisse 40. Durch Verschwenken des drehbar mit dem Bolzen 50 an dem Bock 48 gelagerten Hebels 46 verschiebt dieser die Kulisse 40, so daß die auf der Kulisse 40 ausgebildeten oberen und unteren Halteschlitze 43 bzw. 42, die an der oberen Rahmenhälfte 20 und der unteren Rahmenhälfte 22 angeordneten Haltenasen 44 umlassen. Die schräge Ausbildung der Flanken der oberen Halteschlitze 43 führt zu einer starken Anpressung der oberen Rahmenhälfte 20, deren Kraftwirkung von dem durch den Hebel 46 beim Verschließen beschriebenen Weg abhängt. Dadurch werden die obere Rahmenhälfte 20 und die untere Rahmenhälfte 22 miteinander verriegelt, so daß der Behälterinnenraum 12 allseits verschlossen ist. Selbsthemmung zwischen den Flanken der Halteschlitze 42 bzw. 43 der Kulisse 40 und den Haltenasen 44 bewirkt eine kraftschlüssige Arretierung von Kulisse 40 und gleichzeitig Hebel 46, so daß ein selbsttätiges Öffnen des Behälters oder ein Nachlassen der Verschlußkraft verhindert wird. Durch die in der oberen Rahmenhälfte 20 in der darin ausgebildeten Nut 62 angeordnete Dichtung 60 ist der Behälterinnenraum 12 zudem flüssigkeitsdicht abgedichtet, so daß das den Behälter umgebende Kältemittel nicht in den Behälterinnenraum 12 gelangen kann.

Der so sicher verschlossene Behälter kann nun am Griff 26 ergriffen und in flüssigen Stickstoff zum Gefrieren der in dem Behälter enthaltenen Zellsubstanz getaucht werden.

## Patentansprüche

1. Allseits verschließbarer Behälter, der zum Tieffrieren von in elastischen Beuteln befindlichen Suspensionen lebender Zellsubstanz geeignet ist, gekennzeichnet durch eine an der Außenseite des Behälters mittels einer Klebeschicht befestigte mikroporöse Schicht (30).

2. Behälter nach Anspruch 1, dadurch gekennzeichnet, daß die mikroporöse Schicht (30) aus textilen Materialien besteht.

3. Behälter nach Anspruch 1 oder 2, dadurch gekennzeichnet, daß der Behälter zwei planparallel angeordnete dünnwandige metallische Platten (14, 16), die die Behälterwandung bilden, und einen aus zwei Hälften (20, 22) bestehenden Rahmen aufweist.

4. Behälter nach Anspruch 3, dadurch gekennzeichnet, daß jeweils eine Platte in einer Rahmenhälfte befestigt ist und die beiden Rahmenhälften mittels mindestens eines Gelenkes (52) verschwenkbar aneinander befestigt sind.

5. Behälter nach Anspruch 3 oder 4, dadurch gekennzeichnet, daß die in den Rahmenhälften befestigten Platten durch eine Arretierungsvorrichtung in der planparallelen Stellung arretierbar sind.

6. Behälter nach einem der Ansprüche 3 bis 5, dadurch gekennzeichnet, daß der Behälterinnenraum durch eine in einer Rähmenhälfte angeordnete Dichtung (60) flüssigkeitsdicht abdichtbar ist.

7. Behälter nach einem der Ansprüche 3 bis 6, dadurch gekennzeichnet, daß mindestens eine der Platten mindestens eine Ausnehmung (32; 33) für an dem elastischen Beutel angeordnete Anschlußstutzen oder Pilotröhrchen aufweist.

8. Behälter nach Anspruch 7, dadurch gekennzeichnet, daß die Ausnehmung mit einem Verschlußmittel flüssigkeitsdicht verschließbar ist.

9. Behälter nach einem der Ansprüche 3 bis 8, dadurch gekennzeichnet, daß die Platten (14, 16) auf ihrer zur Behälterinnenseite gerichteten Seite eine Oberflächenrauhigkeit von < 1µm aufweisen.

## Claims

1. Container capable of being closed on all sides, suitable for the deep-freezing of living cellular substances in a suspension in flexible bags, characterised by a micro-porous layer (30) secured to the outside of the container by means of an adhesive coating.

2. Container according to Claim 1, characterised in that the micro-porous layer (20) consists of textile materials.

3. Container according to Claims 1 or 2, characterised in that the container features two thin-walled metallic plates (14, 16), arranged in a parallel plane, which form the container wall, and feature a frame consisting of two halves (20, 22).

4. Container according to Claim 3, characterised in that in each case a plate is secured in one frame half, and the two frame halves are secured to one another by means of at least one joint (52) in a pivoting manner.

5. Container according to Claims 3 or 4, characterised in that the plates secured in the frame halves can be locked in the plane-parallel position by means of a locking device.

6. Container according to Claims 3 to 5, characterised in that the inside of the container is capable of being sealed against liquid by means of a seal (60) arranged in one frame half.

7. Container according to one of Claims 3 to 6, characterised in that at least one of the plates features at least one cut-out (32/33) for the connection nozzles or pilot tubes arranged on the flexible bag.

8. Container according to Claim 7, characterised in that the cut-out is capable of being sealed tight against liquid by means of a closure device.

9. Container according to one of Claims 3 to 8, characterised in that the plates (14, 16) feature a surface roughness of < 1 µm on their side turned towards the inside of the container.

## Revendications

1. Conteneur susceptible d'être verrouillé de toute part, qui convient pour la congélation de suspensions de substances cellulaires vivantes contenues dans des poches élastiques, caractérisé par une couche microporeuse (30) fixée à l'aide d'une couche de colle sur la paroi extérieure du conteneur.

2. Conteneur selon la revendication 1, caractérisé en ce que la couche microporeuse (30) est réalisée dans des matières textiles.

3. Conteneur selon la revendication 1 ou 2, caractérisé en ce que le conteneur comporte deux plaques (14, 16) métalliques minces disposées de manière plane et parallèle, qui forment les parois du conteneur, et un cadre formé de deux moitiés (20, 22).

4. Conteneur selon la revendication 3, caractérisé en ce que chaque plaque est fixée dans une moitié de cadre et les deux moitiés de cadre sont montées l'une contre l'autre de manière à pouvoir pivoter, à l'aide d'au moins une articulation (52).

5. Conteneur selon la revendication 3 ou 4, caractérisé en ce que les plaques fixées dans les moitiés de cadre sont bloquées par un dispositif de blocage dans leur position plane et parallèle.

6. Conteneur selon l'une quelconque des revendications 3 à 5, caractérisé en ce que le compartiment intérieur du conteneur est rendu étanche par un joint d'étanchéité (60) posé dans l'une des moitiés de cadre.

7. Conteneur selon l'une quelconque des revendications 3 à 6, caractérisé en ce qu'au moins une des plaques comporte au moins une cavité (32 ; 33), destinée à recevoir les ajutages ou les tubes témoin montés sur la poche élastique.

8. Conteneur selon la revendication 7, caractérisé en ce que la cavité peut être fermée hermétiquement par un moyen de fermeture.

9. Conteneur selon l'une quelconque des revendications 3 à 8, caractérisé en ce que les plaques (14, 16) présentent sur la face orientée vers l'intérieur du conteneur une rugosité de surface < 1 µm.
